# EUROPEAN PATENT SPECIFICATION

(11) **EP 1 884 218 B1**
(45) Date of publication and mention of the grant of the patent: **25.04.2012**
(21) Application number: 07113491.0
(22) Date of filing: 31.07.2007
(51) Int. Cl.: A61F 2/01

(54) **Sheath unit for recovering thrombus capture member and thrombus capture catheter unit**
Hülleneinheit zur Abdeckung eines Blutgerinnselfilters und Kathetereinheit zur Erfassung des Blutgerinnselfilters
Gaine pour recouvrir un dispositif de capture de thrombus et cathéter pour le dispositif de capture de thrombus

(30) Priority: 01.08.2006 JP 2006210387
(43) Date of publication of application: 06.02.2008
(73) Proprietor: NIPRO CORPORATION, Kita-ku, Osaka-shi, Osaka-fu, 531-8510 (JP)
(72) Inventor: Isshiki, Takaaki, Tokyo Osaka 157-0066 (JP); Kataishi, Yuichi, Yokohama-shi Kanagawa 236-0032 (JP); Miyagawa, Katsuya, Osaka-shi Osaka 531-8510 (JP); Umegaki, Ryota, Osaka-shi Osaka 531-8510 (JP); Furuta, Norihiko, Osaka-shi Osaka 531-8510 (JP)
(74) Representative: Banzer, Hans-Jörg

(56) References cited:
- WO-A-02/40090
- WO-A-2006/102546
- WO-A1-01/21100
- WO-A2-03/004075
- US-A1- 2004 039 412

## Description

### FIELD OF THE INVENTION

The present invention relates to a sheath for recovering a thrombus capture member and a thrombus capture catheter, which capture a thrombus or the like which is freed when performing a percutaneous angioplasty in a hemadostenosis part of coronary artery, carotid artery, venous graft or the like.

### BACKGROUND OF THE INVENTION

Hitherto, in the hemadostenosis part of coronary artery, carotid artery, venous graft or the like, there is performed a percutaneous angioplasty such as stent and POBA (Plain Old Balloon Atherectomy). This operation is one of circulation reconstruction surgeries for ensuring flow of blood by performing a stent detention or a balloon expansion in the hemadostenosis part, and achieves a desired result.

However, in the above method, although the constriction part is expanded, a plaque (torose pathological piece), thrombus or the like, which is freed during the operation, flows to a lesion part periphery side, and thus induces an infarction, a No-Reflow phenomenon or the like, so that there is the fact that re-perfusion is not obtained. And, it is frequent that a complication induced by such thrombus or the like is a critical one.

For example, in an unstable angina pectoris, an acute cardiac infarction or the like, to which the thrombus in the coronary artery relates, there is the fact that the thrombus is pushed out by the stent detention or the balloon expansion, so that a blood vessel in the lesion part periphery side is occluded. As a result, sufficient oxygen and nutrition are not supplied to a cardiac muscle, thereby inducing a cardiac muscle disorder such as cardiac muscle necrosis. Further, similarly, if a blood vessel supplying blood to a brain clogs, a so-called cerebral infarction is induced, and brain tissue dies.

Especially, the carotid artery is liable to generate a constriction by an arterial sclerosis, so that the blood flow to the brain becomes insufficient, and a cerebral infarction is liable to occur by the thrombus formed in the constriction part. As a symptom of such cerebral infarction, there is paralysis, numbness, aphasia, disturbance of consciousness, or the like, and as to a function once lost it is impossible to expect a recuperation of 100% even if rehabilitation is performed, so that a great influence is exerted on a patient' s life thereafter. Further, since a large amount of thrombus adheres to an old transformed venous graft constriction part, caution is required.

Like this, since the infarction caused by the thrombus induces the critical complication, and protection is required in the lesion part periphery side in percutaneous angioplasty, there is already a patent application for a catheter possessing a thrombus capture member (e.g., refer to EP 1 402 826 A2).

WO 02/40090 A1 discloses a thrombus capture catheter unit according to the preamble of claim 1.

### SUMMARY OF THE INVENTION

When recovering the capture member for capturing thrombus and plaque described in EP 1 402 826 A2, as the sheath unit for recovering the capture member there is adopted a sheath whose diameter is the same as the sheath used for delivery or larger than the sheath used for deliverry. Here, in a case where a sheath is used having a diameter approximately the same as the sheath for delivery, although it can reach a distal lesion similar to the sheath concerned, since the capture member which captures the thrombus or the like is required to undergo, at the time of recovery, a change of shape in the sheath to have the compressed form as before expansion, there is a possibility that, before the capture member is from the body, the capture member is pressed, and a captured object leaks into the blood vessel from a pore of the filter of the capture member.

On the other hand, in a case where there is used a sheath whose diameter is larger than the sheath used for delivery, although the capture member with the captured thrombus can be accommodated in the sheath without undergoing the stress of compression a bore diameter in a tip side also becomes large, and such issues occur that reaching the distal lesion which the sheath for delivery can reach becomes difficult, that the sheath becomes liable to undergo an influence of shaft bias in a curred portion of the blood vessel and thus is caught by a stent strut, and that an inner wall of a curved blood vessel is damaged or injured.

Whereupon, there is desired a sheath unit for recovery, in which a compression stress is not exerted on the capture member after recovering a thrombus, and additionally whose diameter is the same as the diameter of the sheath for delivery of the capture member.

In view of the above issues, objects of the present invention are to provide a sheath unit for recovering a thrombus capture member, in which the thrombus capture member can be accommodated without leakage of the thrombus, and which can be more smoothlypassed though a lesion, and to provide a thrombus capture catheter unit which captures freed thrombus and the like.

According to the invention, these objects are achieved by a thrombus capture catheter unit as defined in claim 1. The dependent claim defines preferred and advantageous embodiments of the invention.

The invention concerned with a first aspect of the invention is a thrombus capture catheter unit including a sheath unit having a sheath having a lumen penetrating from its base end to its tip, a flexible shaft inserted so as to be capable of advancing and retreating in an inside of the sheath unit, and a thrombus capture member provided at a tip portion of the shaft and inserted so as to be capable of entering into the tip portion of the sheath from the tip, wherein the thrombus capture member includes a spindle shape, crossed wire member whose tip and base ends, in which plural wires spirally cross, each converge and whose intermediate portion bulges, and a parachute-like filter part in which a filter material is cover-fixed to ( i.e., covers) a distal side of the crossed wire member corresponding approximately to half of the spindle shape, and the sheath is a sheath for recovering a thrombus capture member having a constitution in which a tip flexible tube is provided in a tip part of the sheath, and, when the thrombus capture member is accommodated into the sheath, the crossed wire member constituting the thrombus capture member and a portion of the filter part are accommodated in the tip flexible tube, thereby reducing the diameter of an opening part of the filter part.

According to the first aspect of the invention having the above constitution, since the diameter-reduced part for deflating the opening part of the filter part which captures the thrombus or the like has flexibility, when the thrombus capture member is accommodated into the sheath unit, the thrombus capture member is not pressed and the captured object does not leak into the blood vessel from the pores of the filter. Additionally, it is possible to deflate the opening part of the filter part bythediameter-reducedpart having flexibility, and it is possible to obtain a thrombus capture catheter unit which can certainly accommodate the accommodated object without leaking.

The invention concerned with a second aspect of the invention is characterized by having a constitution in which the thrombus capture member possesses in its tip (or distal) end a slide ring and possesses in its base (or proximal) end a fixed ring, and when the sheath is slid to a distal side a stopper provided in the sheath unit is near the fixed ring and the crossed wire member constituting the thrombus capture member and one part of the filter part are accommodated in the tip flexible tube.

According to the second aspect of the invention having the above constitution, it is possible to always accommodate the thrombus capture member in a accurate position, and it is possible to accommodate the thrombus capture member without pressing it more than necessary, under a state of having no stress, and without leakage of the accommodated object.

### BRIEF DESCRIPTION OF THE DRAWINGS

Fig. 1 is a plan view showing one example of a sheath unit for recovering a capture member according to the present invention.
Fig. 2 is a plan view showing another embodiment of the sheath unit for recovering the capture member.
Fig. 3 is a sectional view showing various shapes of the tip flexible tube.
Fig. 4 is a sectional view showing various shapes of the tip flexible tube.
Fig. 5 is a sectional view showing various shapes of the tip flexible tube.
Fig. 6 is a plan view showing an action state of the sheath unit for recovering the capture member.
Fig. 7 is a plan view showing an action state of another implementationmode of the sheath unit for recovering the capture member according to the present invention.

### THE MOST PREFERRED EMBODIMENTS OF THE INVENTION

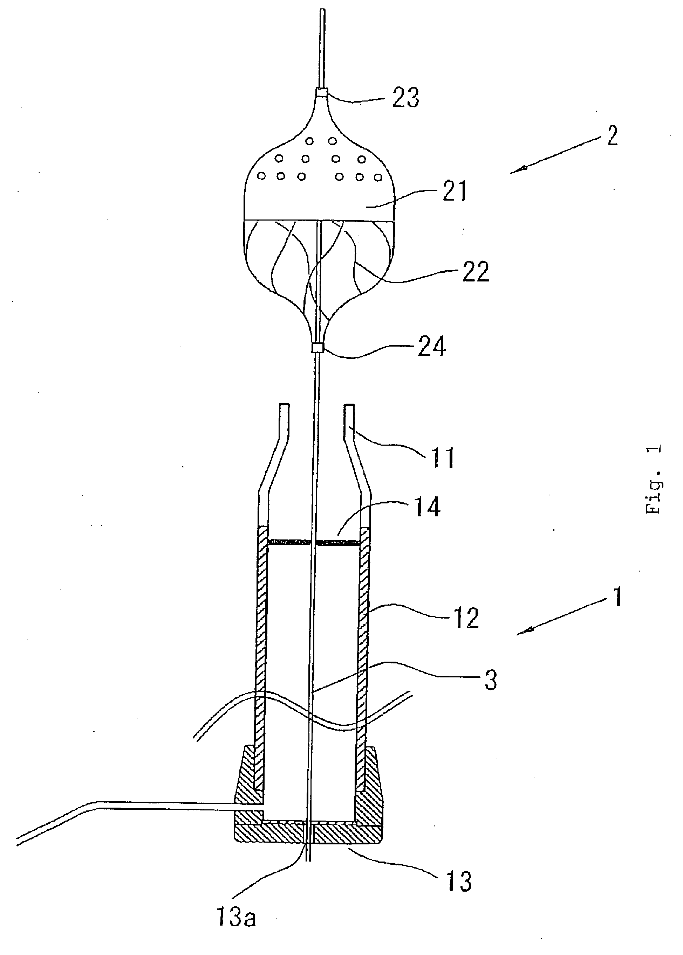

Hereunder, implementation modes of a sheath unit for recovering the thrombus capture member and a thrombus capture catheter unit according to the present invention are explained in detail on the basis of Fig. 1 to Fig. 7.

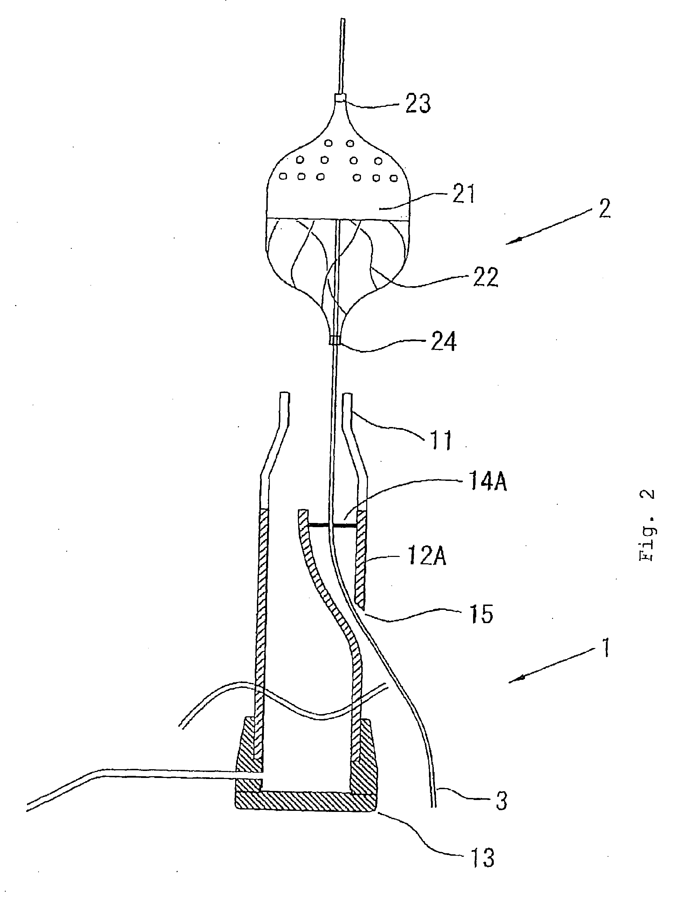

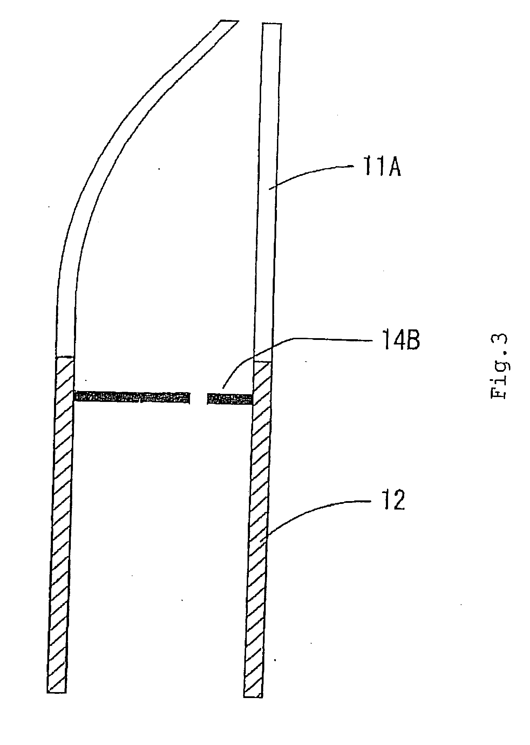

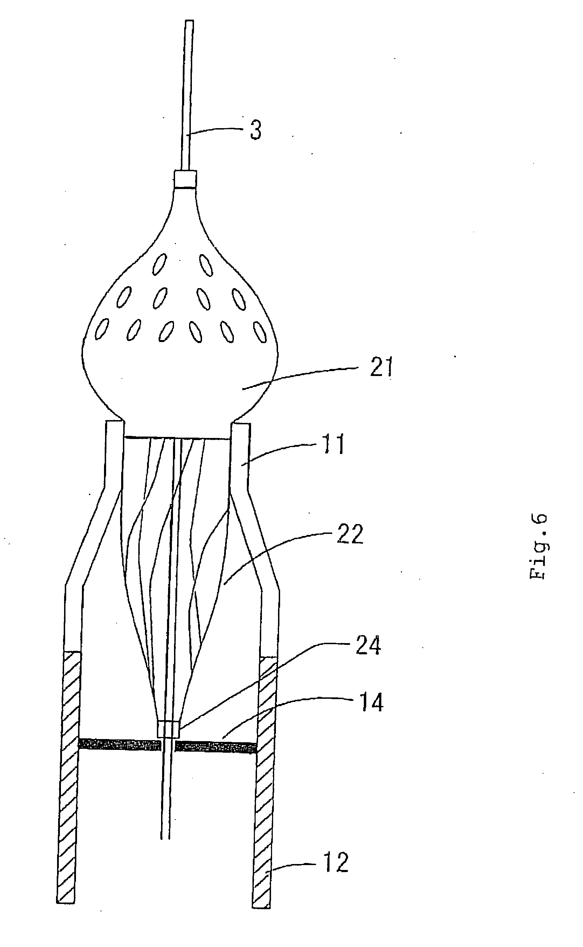

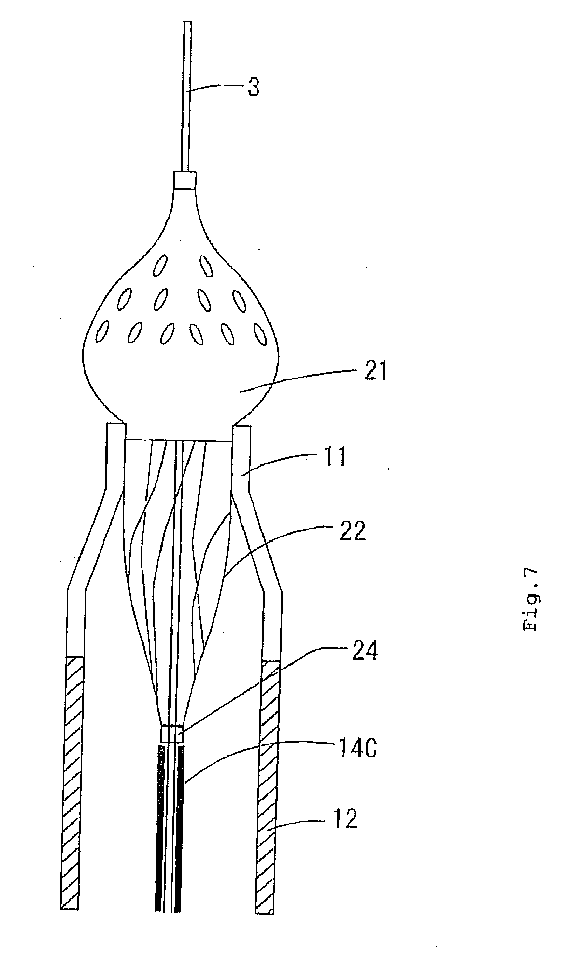

Fig. 1 is a plan view showing one example of the sheath unit for recovering the thrombus capture member according to the present invention. Fig. 2 is a plan view showing another embodiment of the sheath unit according to the present invention. Fig. 3 - Fig. 5 are sectional views showing various shapes of a tip flexible tube. Fig. 6 is a plan view showing an action state of the sheath unit for recovering the thrombus capture member according to the present invention, and Fig. 7 is a plan view showing an action state of another implementation mode of the sheath unit for recovering the thrombus capture member according to the present invention.

A sheath unit 1 for recovering a thrombus capture member, which is shown in Fig. 1, is constituted by a tip flexible tube 11, a sheath 12, and an occlusion member 13 in a base end part.

Further, a stopper 14 is provided inside the sheath unit. Since it is important that the sheath 12 is a biocompatibile member having flexibility, it is a hollow tubular member comprising a synthetic resin such as, e.g., polyurethane, polyethylene, polyester, polypropylene, polyamide, polytetrafluoroethylene, and polyvinylidene fluoride.

A thrombus capture member 2 is provided outside of the tip flexible tube 11 of sheath unit 1 and, through a shaft 3 inserted through a lumen of the sheath unit 1 for recovering the thrombus capture member, there is constituted a thrombus capture catheter unit which is capable of accommodating the thrombus capture member 2 in the tip flexible tube 11.

The thrombus capture member 2 is inserted into a tip portion of the tip flexible tube 11 so as to be capable of entering into the tip, and includes a filter part 21 for capturing the thrombus and a crossed wire member 22 resembling a net bag, in which plural wires spirally intersect or cross. The crossed wire member 22 has a shape in which its tip and base ends, which are made with the plural wires being spirally crossed, each converge and its intermediate portion bulges outwardly and, on a distal side including the bulged intermediate part of this crossed wire member, there is provided filter part 21. Here, the diameter of the pores of the filter ranges from 50 µm1000 µm, desirably 50 µm - 500 µm, and most desirably 100 µm - 200 µm. If the pore diameter of the pores of the filter part is less than 50 µm, flow of blood is hindered and, if it exceeds 1000 µm, the filter part has no utility.

The thrombus capture member 2 has at its tip or distal end a slide ring 23 and has at its base end a fixed ring 24 and, by means of the slide ring 23 and the fixed ring 24, its tip is slidably attached to the shaft 3 and its base end is fixed to the shaft 3. And, when the sheath unit 1 for recovering the thrombus capture member is slid in a distal direction in regard to the thrombus capture member 2, there are accommodated in the sheath unit 1 the crossed wire member 22 constituting the capture member and a portion of the filter part 21.

The stopper 14 is a stopper regulating sliding of the fixed ring 24 that is a fixed part of the crossed wire member 22, and it is desirable that it is formed of a biocompatible synthetic resin havingflexibilitysuch as,e.g.,polyurethane, polyethylene, polyester, polypropylene, polyamide, polytetrafluoroethylene, and polyvinylidene fluoride.

As a material of the wire of the crossed wire member 22, a shape memory alloy can be adopted, and generally there is adopted a Ni-Ti system alloy, a Cu-Zn-Al system alloy, or the like. In a case where the shape memory alloy is adopted as the material forming the wire, usually the crossed wire part 22 is memorized in a shape (reversion shape) as shown in Fig. 1. In order to be applied to a lesion part in which there are many thrombi or the like, it is also possible to form a longer crossed wire member so as to be capable of capturing more of the thrombis. Further, the number of wires constituting the crossed wire member 22 is 8 - 16, desirably 8 - 14, and most desirably 8 - 12.

Incidentally, as an outer diameter of the crossed wire member 2, there is adopted, although not limited especially, an outer diameter of 3.0, 3.5, 4.0, 4.5, 5.0, 5.5, 6.0, 6.5, 7.0, 7.5, or 8.0 mm, and it is desirable to selectively choose it in compliance with a blood vessel diameter in the lesion part.

It is desirable that the filter material is one which can be dissolved to form a solution into which the crossed wire member 22 can be immersed. Desirably, there is enumerated a synthetic resin of a biocompatible material such as, e.g., polyurethane, polyethylene, polyester, polypropylene, polytetrafluoroethylene, or polyvinylidene fluoride.

The occlusion member 13 is a member closing the base end part of the sheath unit 1 for recovering the thrombus capture member, and is a tubular member capable of being formed from a plastic such as polypropylene, ABS resin, polyvinyl chloride, polyethylene, and polyethylene terephthalate, or a metal such as stainless steel and brass.

Although a base end of the occlusion member 13 is closed, a hole 13a along an axis is provided such that the shaft 3 can be inserted.

Further, as shown in Fig. 2, it is also possible to make a constitution in which a side hole 15 into which the shaft 3 can be inserted is provided in a tip side side-wall of the sheath. In this example, there is made a sheath 12A possessing a second lumen into which the thrombus capture member 2 can be inserted, and a stopper 14A is provided in this second lumen part.

The tip flexible tube 11 is provided on a sheath tip, and it is desirable that it is formed desirably of a biocompatible synthetic resin havingflexibilitysuch as,e.g.,polyurethane, polyethylene, polyester, polypropylene, polyamide, polytetrafluoroethylene, and polyvinylidene fluoride. The tube length of the tip flexible tube 11 that is a tubular member having a lumen is, although not limited especially, 1.0 - 50 mm, desirably 1 - 25 mm, and most desirably in the order of 5 - 15 mm.

The tip flexible tube 11 is made into a shape in which its tip part is diameter-reduced, and acts so as to slightly deflate an opening part of the filter part 21 that is opened like a parachute, when the fixed ring 24 is stopped by abutting against the stopper 14 when the thrombus capture member 2 is accommodated into the sheath unit 1 for recovering the thrombus capture member. Therefore, it is possible to obtain the sheath unit 1 for recovering the thrombus capture member, in which there is no possibility that the filter part 21 for capturing the thrombus or the like is pressed strongly, and there is no possibility that the captured object leaks into the blood vessel from the pores of the filter. Additionally, if there is made a catheter having the tip flexible tube 11, the lesion part passing ability in the blood vessel is raised, and it is possible to obtain a thrombus capture catheter unit in which a certain recovery of the recovered, captured object is possible.

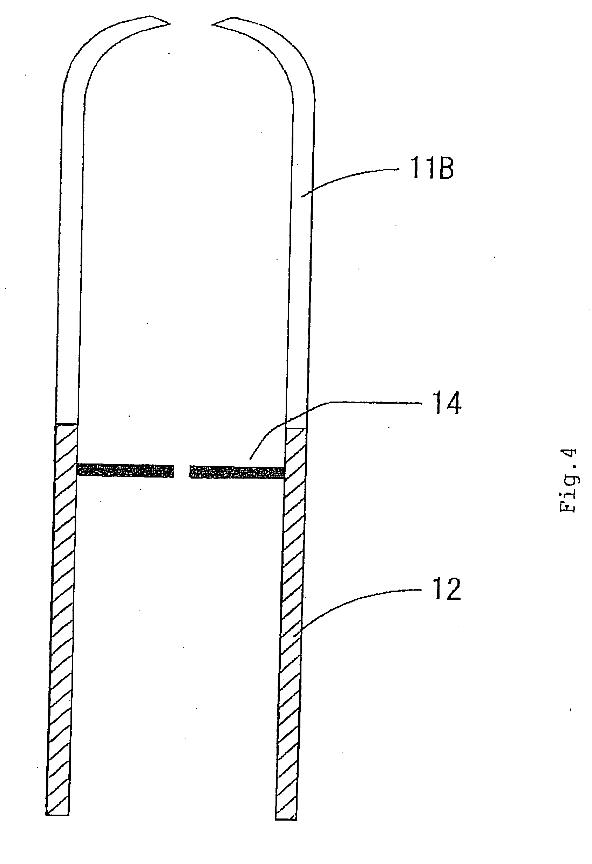

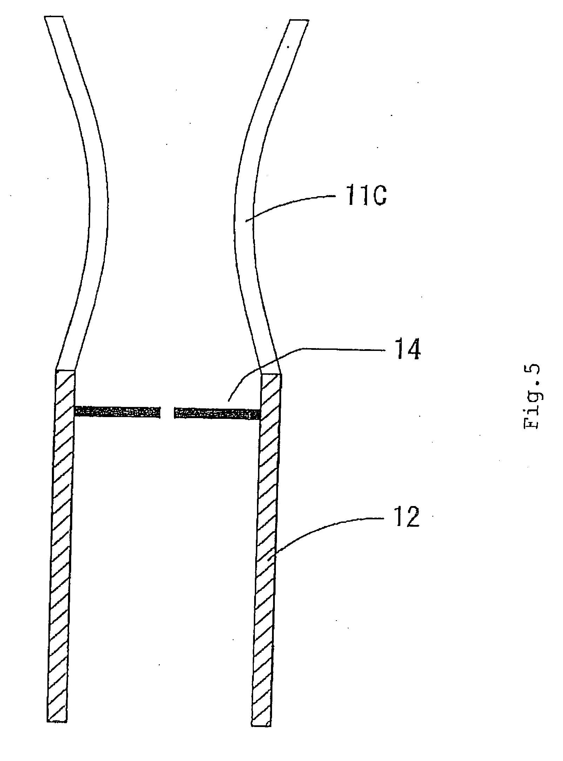

Further, as to the tip flexible tube 11, it is important that it have a shape in which the filter part 21 for capturing the thrombus can be accommodated without stress, and that the tip is diameter-reduced. Therefore, its opening may deviate from an axis of the lumen as shown in Fig. 3, or there may be such a shape having a circular opening in its tip as shown in Fig. 4, or such a shape in which an intermediate portion of the tube is diameter-reduced as shown in Fig. 5. In a case of a tip flexible tube 11A as shown in Fig. 3, which has an eccentric opening part whose center deviates from the axis of the lumen, there is adopted a stopper 14B whose opening is similarly provided in an eccentric position. Further, in a case of a tip flexible tube 11B as shown in Fig. 4, having a circular opening in its tip, or a tip flexible tube 11C shown in Fig. 5 having a shape in which an intermediate portion of the tube is diameter-reduced, it is possible to use a stopper 14 having an opening part in its center.

Next, referring to Fig. 6 and Fig. 7, there is explained an action state of the sheath unit for recovering the capture member.

Fig. 6 illustrates a method of recovering (not part of the invention), after percutaneous angioplasty, the thrombus capture member, which has captured a thrombus or the like, into the sheath unit for recovering. By sliding the sheath unit 1 for recovering the capture member, through which the shaft 3 is inserted, to the distal side, the intersection wire member 22 constituting the thrombus capture member 2 and a portion of the filter part 21 can be accommodated in the tip flexible tube 11.

When accommodating the thrombus capture member, most of the filter part 21 exists outside of the tip flexible tube 11, and the captured object is not pressed. Further, the stopper 14 provided in the sheath unit regulates, in an inside of the sheath unit, an excessive sliding of the fixed ring 24, that is the fixed part of the crossed wire member, to a base end side without impeding the sliding of the shaft 3. By this slide regulation, an accommodation position of the thrombus capture member 2 can be singly determined.

As mentioned above, it is desirable that the stopper 14 is formed of a biocompatible synthetic resin having flexibility. Further, its shape is not limited to the disc-like stopper 14 in the sheath unit, and it is also possible to use the above-mentioned stoppers 14A, 14B, or a stopper 14C whose part against the fixed ring 24 is tubular as shown in Fig. 7.

Accordingly, there is provided a constitution in which, by using the tip flexible tube whose tip opening portion is diameter-reduced, when the thrombus capture member is accommodated in the sheath unit and a stop position of the member is regulated by the stopper, the tip flexible tube and the crossed wire member and the filter portion, which are mutually flexible, about, and thus the latter two are accommodated at predetermined accurate positions while being elastically deformed. Therefore, it follows that, in the inside of the sheath unit, the intersection wire member 22 and a portion of the filter part 21 are correctly accommodated in the predetermined positions, and there can be provided a constitution in which an accommodated object, such as a thrombus, captured by the filter part 21 is neither squeezed by the pores of the filter nor leaks.

As described above, since the sheath unit for recovering the capture member, which is concerned with the present invention, has a constitution in which the tip flexible tube is provided on the tip part of the sheath, and the crossed wire member constituting the thrombus capture member and a portion of the filter part are accommodated in the tip flexible tube when the thrombus capture member is accommodated in the sheath, thereby diameter-reducing the opening part of the filter, the thrombus capture member can be recovered in the diameter-reduced portion of the sheath unit. Further, since the opening part of the filter which captures the thrombus or the like is deflated and no stress is applied thereto, there is obtained a sheath unit for recovering a thrombus capture member, which can remove the captured thrombus from the body without leaking.

Therefore, since there is provided a thrombus capture catheter unit in which a sheath unit for recovering the thrombus capture member and the thrombus capture member are combined, the capture member taking in the thrombus can be recovered in a fine diameter sheath, and it becomes possible to remove the capturedthrombus from the body without leaking. Additionally, since there is possessed the tip flexible tube, a lesion part passing ability in the blood vessel is increased, and a certain recovery of the recovered, captured object becomes possible. Further, by this fact, it is possible to reduce a patient's physical and psychological burdens.

### INDUSTRIAL APPLICABILITY

According to the present invention, it is possible to recover a thrombus capture member, in which thrombus was captured, by a fine diameter sheath, and it becomes possible to remove the captured thrombus from the body without leaking. Further, by this fact, it is possible to reduce a patient's physical andpsychologicalburdens. Additionally, alesionpartpassing ability in the blood vessel in which the catheter unit is used is raised, and a certain or sure recovery of the recovered, captured object becomes possible.

## Claims

1. A thrombus capture catheter unit comprising a sheath unit (1) comprising a sheath (12; 12A) having a lumen penetrating from a base end to a tip end of the sheath (12; 12A), a flexible shaft (3) inserted in the lumen so as to be capable of advancing and retreating in an inside of the sheath (12; 12A), and a thrombus capture member (2) inserted so as to be capable of entering into a tip portion of the sheath (12; 12A), wherein the thrombus capture member (2) comprises a parachute-like filter part (21) in which a filter material covers an end part of the wire member (22), **characterized in that** the thrombus capture member is provided at a tip portion of the shaft (3) and comprises a spindle shape crossed wire member (22) comprising a plurality of spirally arranged crossing wires converging at tip and base ends of the wire member (22) and bulging in an intermediate portion of the wire member (22), the said end part of the wire member covered by the said filter material corresponds to about a half of the spindle shape wherein, when the thrombus capture member (2) is accommodated into a flexible tube (11; 11B; 11C) of the sheath unit (1), the crossed wire member (22) of the thrombus capture member (2) and a portion of the filter part (21) are accommodated in the flexible tube (11; 11B; 11C), thereby diameter-reducing an opening end of the filter part (21).

2. A thrombus capture catheter unit according to claim 1, wherein the thrombus capture member has at its distal end a slide ring (23) and has at its base end a fixed ring (24), and wherein a stopper (14; 14A; 14B; 14C) is provided in the sheath (12; 12A) and when the sheath (12; 12A) is slid in a distal direction the stopper (14; 14A; 14B; 14C) is near the fixed ring (24) and the crossed wire member (22) constituting the thrombus capture member (2) and a portion of the filter part (21) are accommodated in the tip flexible tube (11; 11B; 11C).

## Patentansprüche

1. Kathetereinheit zum Erfassen eines Blutgerinsels, umfassend eine Hülleneinheit (1), umfassend eine Hülle (12; 12A) mit einem Hohlraum, der von einem Basis-Ende zu einem Spitze-Ende der Hülle (12; 12A) vordringt, einen elastischen Schaft (3), der in den Hohlraum derart eingeführt ist, dass er in ein Inneres der Hülle (12; 12A) vordringen und sich daraus zurückziehen kann, und ein Blutgerinsel-Erfassungsteil (2), das derart eingeführt ist, dass es in einen Spitzen-Abschnitt der Hülle (12; 12A) eintreten kann, wobei das Blutgerinsel-Erfassungsteil (2) ein fallschirmartiges Filterteil (21) umfasst, in dem ein Filtermaterial ein Endteil des Drahtteils (22) abdeckt, **dadurch gekennzeichnet, dass** das Blutgerinsel-Erfassungsteil an einem Spitzen-Abschnitt des Schafts (3) bereitgestellt ist und ein spindelförmiges Teil (22) mit gekreuzten Drähten umfasst, welches eine Vielzahl von spiralförmig angeordneten gekreuzten Drähten umfasst, die an den Spitzen- und Basis-Enden des Drahtteils (22) zusammenlaufen und sich in einem Zwischenabschnitt des Drahtteils (22) auseinanderwölben, wobei das Endteil des von dem Filtermaterial abgedeckten Drahtteils ungefähr einer Hälfte der Spindelform entspricht, wobei, wenn das Blutgerinsel-Erfassungsteil (2) in einem elastischen Rohr (11; 11B; 11C) der Hülleneinheit (1) aufgenommen ist, das Teil (22) mit gekreuzten Drähten des Blutgerinsel-Erfassungsteils (2) und ein Abschnitt des Filterteils (21) in dem elastischen Rohr (11 ; 11B ; 11C) aufgenommen sind, wodurch der Durchmesser eines öffnenden Endes des Filterteils (21) reduziert wird.

2. Kathetereinheit zum Erfassen eines Blutgerinsels nach Anspruch 1, wobei das Blutgerinsel-Erfassungsteil an seinem distalen Ende einen verrutschbaren Ring (23) hat und an einem Basis-Ende einen befestigten Ring (24) hat, und wobei ein Anschlag (14; 14A; 14B; 14C) in der Hülle (12; 12A) bereitgestellt ist, und wobei, wenn die Hülle (12; 12A) in eine distale Richtung verrutscht wird (14; 14A; 14B; 14C), der Anschlag (14; 14A; 14B; 14C) nahe dem befestigten Ring (24) ist, und wobei das Teil (22) mit gekreuzten Drähten, welches das Blutgerinsel-Erfassungsteil (2) ausbildet, und ein Abschnitt des Filterteils (21) in dem elastischen Rohr (11; 11B; 11C) der Spitze aufgenommen sind.

## Revendications

1. Ensemble cathéter de capture de thrombus comprenant un ensemble gaine (1) comprenant une gaine (12 ; 12A) comportant une lumière pénétrant depuis une extrémité de base jusqu'à une extrémité de pointe de la gaine (12 ; 12A), une tige flexible (3) introduite dans la lumière de manière à pouvoir avancer et reculer à l'intérieur de la gaine (12 ; 12A), et un élément de capture de thrombus (2) introduit de manière à pouvoir pénétrer dans la partie pointe de la gaine (12 ; 12A), dans lequel l'élément de capture de thrombus (2) comprend une pièce formant filtre en forme de parachute (21) dans laquelle un matériau filtrant couvre une partie extrémité de l'élément formant fil (22), **caractérisé en ce que** l'élément de capture de thrombus est prévu dans une partie pointe de la tige (3) et comprend un élément formant fil croisé en forme de fuseau (22) comprenant une pluralité de fils croisés agencés en spirale, qui convergent aux extrémités de pointe et de base de l'élément formant fil (22) et sont bombés dans une partie intermédiaire de l'élément formant fil (22), ladite partie extrémité de l'élément formant fil couverte par ledit matériau filtrant correspond à environ la moitié de la forme en fuseau, dans lequel, lorsque l'élément de capture de thrombus (2) loge à l'intérieur d'un tube flexible (11 ; 11B ; 11C) de l'ensemble gaine (1), l'élément formant fil croisé (22) de l'élément de capture de thrombus (2) et une partie de la pièce formant filtre (21) logent dans le tube flexible (11 ; 11B ; 11C), réduisant ainsi le diamètre d'une extrémité d'ouverture de la pièce formant filtre (21).

2. Ensemble cathéter de capture de thrombus selon la revendication 1, dans lequel l'élément de capture de thrombus comporte, à son extrémité distale, un anneau coulissant (23) et comporte, à son extrémité de base, un anneau fixe (24), et dans lequel un bouchon (14 ; 14A ; 14B ; 14C) est prévu dans la gaine (12 ; 12A) et lorsque la gaine (12 ; 12A) coulisse dans le sens distal, le bouchon (14 ; 14A ; 14B ; 14C) est près de l'anneau fixe (24) et l'élément formant fil croisé (22) constituant l'élément de capture de thrombus (2) et une partie de la pièce formant filtre (21) logent dans le tube flexible de pointe (11 ; 11B ; 11C).
